# EUROPEAN PATENT SPECIFICATION

(11) **EP 0 246 680 B1**
(45) Date of publication and mention of the grant of the patent: **23.06.1993**
(21) Application number: 87200709.1
(22) Date of filing: 15.04.1987
(51) Int. Cl.: A61K 39/295

(54) **Combined vaccine**
Kombinierter Impfstoff
Vaccin combiné

(30) Priority: 21.04.1986 NL 8601001
(43) Date of publication of application: 25.11.1987
(73) Proprietor: Akzo Nobel N.V., 6824 BM Arnhem (NL)
(72) Inventor: Lütticken, Heinrich Dieter, NL-5831 CE Boxmeer (NL); Visser, Nicolaas, NL-5831 RV Boxmeer (NL); Rijke, Eric Onno, NL-5835 AM Beugen (NL)
(74) Representative: Hermans, Franciscus G.M.

(56) References cited:
- EP-A- 0 115 442
- FR-A- 2 569 984
- GB-A- 1 401 565
- INFECTION AND IMMUNITY, vol. 37, no. 2, August 1982, pp. 586-591, Washington, US; D.R. SNODGRASS et al.: "Passive immunity in calf diarrhea: vaccination with K99 antigen of enterotoxigenic Escherichi coli and Rotavirus"
- M. DIXON et al.: "Enzymes", 2nd Edition, 1971, pp. 145-150, Longman, London, GB

## Description

The invention relates to a combined vaccine and also to a method for the preparation of such a vaccine and to the use of such a vaccine.

In commercial stock-breeding the role of vaccination for the maintenance of a healthy livestock population is particularly important.

This applies in particular also to intensive stock-breeding with associated high population densities, where a viral, bacterial or parasitic infection can affect the whole stock in a short time - often with disastrous consequences.

To prevent infection livestock are therefore vaccinated, for example, separately against, inter alia, Escherichia coli (E.coli) infections, and against herpes virus infections, such as pseudorabies (also termed Aujeszky's disease).

Said vaccinations are usually carried out by administering immunogenic material in the form of live (attenuated or unattenuated) pathogens, or by means of killed pathogens.

It is obvious that from the economic point of view it is attractive to prepare immunogenic material from various pathogens combined as one vaccine and to administer it as such.

It was found not to be possible, however, to prepare a combined vaccine which has a stable immunizing activity both against disorders due to E.coli and against disorders due to herpes virus by simply adding fresh E.coli material and viral material together. Surprisingly, it has also been found, however, that such a combined vaccine can in fact be prepared.

The object of the present invention is therefore to provide a combined vaccine with which immunization can be carried out simultaneously against E.coli and against herpes virus.

More particularly, such a vaccine contains immunogenic E.coli material and viral material in which undesired enzyme activity is inhibited or is largely absent.

Surprisingly, it has been found in particular that the immunogenic activity of the viral material present in the combined vaccine is substantially improved if the enzyme activity is suppressed or removed prior to, during and/or after the combination of the two vaccine components.

Probably, this concerns in particular the activity of enzymes present in the E.coli material. Without being able to advance an explanation for this with certainty, the supposition is that, in particular, proteolytic enzymes from the E.coli material might adversely affect the immunogenic potency of the combined vaccine.

The E.coli material, and if desired also the virus material, can be rendered free or virtually free of enzyme activity prior to the preparation of the combined vaccine. This may be performed actively or passively.

The enzyme activity may be removed passively by storing the E.coli material for the preparation of the combined vaccine separate for at least a sufficiently long time. Sufficient is, for example, approximately two to four weeks at approximately 4 °C. In the light of the possible explanation advanced above it could be concluded that as a result of this an ageing process occurs in which the proteolytic enzymes are inactivated. It is possible to actively render free from enzyme activity, for example, by first largely free the separate materials from enzyme activity, for example by precipitation, (column) chromatography, centrifuging, electrophoresis, or other biochemical or microbiological separation and purification methods or by adding enzyme inhibitors, by keeping the materials for a sufficiently long period, by adding high concentrations of foreign protein (for example in the form of serum or serum proteins), or by denaturing the enzymes.

As an alternative, or in addition, the enzyme activity can be markedly or completely reduced during or immediately after the preparation of the combined vaccine, for example, by adding enzyme inhibitors or high concentrations of foreign protein, or by separating the enzyme activity from the immunogenic materials by means of biochemical or microbiological separation methods.

The invention relates in particular to combined vaccines in which herpes viruses such as pseudorabies virus or bovine herpes virus are incorporated.

Suitable enzyme inhibitors are, for example: N-ethylmaleimide, monoiodoacetate, monoiodoacetamide, Trasylol, EDTA, PMSF and trypsin inhibitors such as the so-called "soybean trypsin inhibitor".

Suitable proteins for adding to the vaccine or the vaccine components are, for example, the serum proteins, already mentioned earlier, in the form of total serum or certain components thereof (such as, for example, the serum albumins) or milk proteins or egg proteins.

In the vaccines according to the invention the isolated antigen fraction of E.coli or E.coli variants (spontaneous or induced mutants, or E.coli modified by recombinant DNA techniques) is used. For pig vaccines this refers, for example, to an antigen fraction which contains the so-called K88-pili antigen. Of course, E.coli antigens may be used which have been prepared by a different microorganism, the genetic material of which has been modified by means of recombinant techniques in a manner such that E.coli antigens can be produced if desired with increased yield.

The herpes viral material for the vaccine according to the invention comprises killed or live, if desired attenuated, virus, the pathogenicity and/or the virulence of which is reduced or destroyed optionally by spontaneous or induced mutations or by recombinant DNA techniques. And it is optionally also possible to use isolated immunogenic viral material in the combined vaccine.

It is found to be possible, for example, to use such a combined vaccine according to the invention for the simultaneous immunization of pigs to protect their off-spring both against pig diarrhoea and against pseudorabies viral infections.

A vaccine according to the invention may, if desired, contain the following components in addition to the immunogenic material:
- stabilizers;
- adjuvants, such as aluminium salts (for example Al(OH)₃; AlPO₄, Al₂(SO₄)₃); Ca₃(PO₄)₂; saponin; DDA; Pluronics; avridin; oil-in-water emulsions, if desired together with vitamin E, Pluronics, avridin, dextran sulphate or the like: water-in-oil emulsions, if desired with Marcol, Polysorbate 80, polysorbitan monooleate, saponin, miglyol, isopropyl myristate, isopropyl palmitate or the like, or in the form of capsules of, for example, gelatin or hydroxypropylmethylcellulose phthalate with or without saponin;
- buffers such as phosphate buffer, bicarbonate buffer or tris buffer, preferably in a strength of 5-100 mmol/l;
- preservatives such as Thiomersal, m- or o-cresol or formalin (preferably in a quantity of 0.2 - 0.5%) or benzyl alcohol (preferably 1-2%).

The invention is explained by reference to the following examples.

### Example 1

In order to establish the negative effects of untreated E.coli antigens on pseudorabies virus, the infectivity of the pseudorabies virus was measured in the presence of an equal volume of the E.coli antigens.

E.coli antigens were prepared as follows. E.coli's were cultured in casein hydrolysate/sorbitol media under standard conditions with continuous adjustment of pH, oxygen and stirring intensity in a fermentator.

After 40 hours the culture was stopped and the content of the fermentator was heated for 15 minutes at 60-65 °C. After cooling down the material was centrifuged. The antigens in the supernatant were concentrated by ultrafiltration. The resulting antigen concentrate was incubated for 16 hours at 37 °C in the presence of 0.5% formaline.

After incubating the freshly prepared E.coli antigen with pseudorabies virus for 1 hour at 37 °C, the virus was titered out in 10-fold dilutions into microtiter plates on monolayers of Vero cells. The virus titer was calculated from the CPEs found.

The results in the table show that the E.coli material has an appreciable effect on the infectivity of the pseudorabies virus.

| No. | Pseudorabies virus (ml) | E.coli antigens | | Titer (log¹⁰/ml) | ΔTiter compared with control |
|---|---|---|---|---|---|
| | | ml | type | | |
| 1 | 0.5 | -- | -- | 5.27 | -- |
| 2 | 0.5 | 0.5 | K88ab | 3.05 | 2.22 |
| 3 | 0.5 | 0.5 | K88ac | 2.53 | 2.74 |
| 4 | 0.5 | 0.5 | K99 | 4.79 | 0.48 |

### Example 2

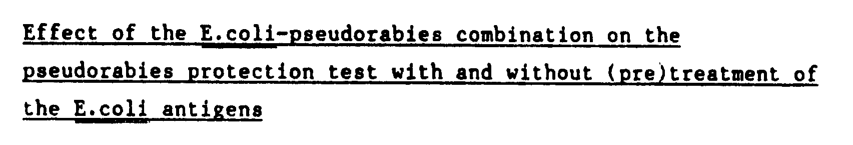

This example demonstrates that without the treatments according to the invention, the combination of pseudorabies antigen and E.coli antigens does not result in an efficient vaccine combination. The effectiveness of the pseudorabies antigens was measured in a mouse protection test.

The mice (C57 b110 strain) were vaccinated with a decreasing quantity of vaccine (100, 25 or 6.25 µl respectively) and after 4 weeks a test infection was carried out with the virulent pseudorabies virus strain, Phylaxia. From this the dose was calculated which gives 50% protection (PD₅₀). From the experiments it is evident that without treatment of the E.coli antigen the vaccine does not satisfy the quality requirements for pseudorabies vaccine (PD₅₀ ≦ 50 µl).

After treatment according to the invention the combined vaccine does in fact satisfy said quality requirement.

| Batch No. | Pseudorabies antigen | E.coli LTK88 | Treatment | PD₅₀ (in µl) | Notes |
|---|---|---|---|---|---|
| 3592 | 40% | 5% | none | >150 | rejected (≧50) |
| 3633 | 40% | - | none | 25.0 | good |
| 4508 | 41% | 4% | none | 119.0 | rejected (≧50) |
| 4509 | 41% | - | 10% calf serum | 10.7 | good (≦50) |
| 4510 | 41% | 4% | 10% calf serum | 28.0 | good (≦50) |
| 5087 | 25% | - | none | 12.9 | good (≦50) |
| 5089 | 25% | 12% | 4% calf serum | 40.7 | good (≦50) |
| 5091 | 25% | 12% | 200 units of trasylol | 19.1 | good (≦50) |

### Example 3

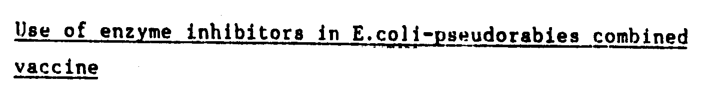

The presumed endogenous protease activities were suppressed by means of a number of enzyme inhibitors. The choice given does not exclude other inhibitors.

The effect of the inhibitors was measured in a standard ELISA for determining antigen quantities.

Without treatment the measurable quantity of pseudorabies antigen is reduced to 53%. As a result of the various treatments at least 93% of the antigen quantity remains measurable in this test.

| No. | pseudorabies antigen | E.coli antigens | Treatment | ELISA result % compared with control (N) |
|---|---|---|---|---|
| 1 | + | - | - | 101.0% ( 5) |
| 2 | - | + | - | 0% ( 3) |
| 3 | + | + | - | 53.0% ( 3) |
| 4 | + | + | PMSF | 92.9% ( 5) |
| 5 | + | + | NMI | 102.9% ( 4) |
| 6 | + | + | MIA | 93.9% ( 3) |
| 7 | + | + | TRAS | 94.5% ( 3) |
| 8 | + | + | TI | 97.5% ( 4) |
| 9 | + | + | EDTA | 95.3% ( 3) |
| 10 | + | + | ageing | 102.5% (10) |
| PMSF : phenylmethanesulphonyl fluoride NMI : N-ethylmaleimide MIA : monoiodoacetamide TI : trypsin inhibitor (soybean) TRAS : Trasylol EDTA : ethylenediamine tetraacetic acid. | | | | |

### Example 4

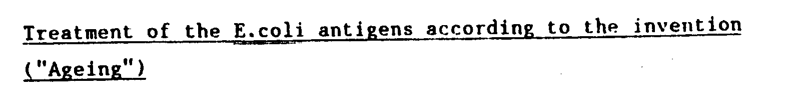

6-8-week-old mice (Swiss albino) were intramuscularly injected with graded dosages (4, 16 or 64 µl respectively) of the specified vaccines. Four weeks after vaccination the mice were given a test infection as in Example 2.

The protection of the mice is expressed in the dose which gives 50% protection (PD₅₀). The quality requirement for approval is that the PD₅₀ should be ≦ 50 µl.

The antibodies for the E.coli components were measured in the sera of the same mice by means of a standard ELISA for the determination of antibody titers. Each figure is the average of 6 sera.

### Conclusions:

The antibody titers for the E.coli components are comparable in the combined vaccine with those of the E.coli vaccine on its own. The protection test against pseudorabies in mice satisfies the quality requirements for such a combined vaccine.

| Batch No. | Pseudorabies antigen | E.coli antigen | PD₅₀ | Anti E.coli titers (16 µl dose) log₂ in ELISA | | | | |
|---|---|---|---|---|---|---|---|---|
| | | | | LT | K88ab | K88ac | K99 | 987P |
| 6704 | + | | 5.4 | | | | | |
| 6705 | + | | 11.9 | | | | | |
| 6706 | + | | 19.5 | | | | | |
| 6707 | | + | | 6.8 | 10.8 | 10.8 | 8.2 | 11.2 |
| 6708 | | + | | 6.5 | 10.8 | 10.2 | 8.8 | 11.5 |
| 6709 | | + | | 7.3 | 11.7 | 12.3 | 10.0 | 11.7 |
| 6710 | + | + | 16.1 | 6.8 | 12.5 | 12.3 | 9.0 | 12.5 |
| 6711 | + | + | 8.9 | 7.5 | 12.2 | 12.2 | 9.5 | 12.2 |
| 6712 | + | + | 23.6 | 7.2 | 11.8 | 11.7 | 9.0 | 12.2 |

## Claims (Claims for the following Contracting State(s): BE, CH, DE, FR, GB, IT, LI, NL, SE)

1. Combined vaccine with immunizing action in animals against E. coli and herpes virus infection obtainable by combining E. coli immunogenic material which is free of enzyme activity with herpes virus immunogenic material.

2. Combined vaccine according to claim 1, characterized in that the vaccine contains an isolated antigen fraction of E. coli.

3. Combined vaccine according to claim 2, characterized in that the antigen fraction contains LT.

4. Combined vaccine according to claim 2, characterized in that the antigen fraction contains pili.

5. Combined vaccine according to claim 4, characterized in that the vaccine comprises one or more pili selected from the group consisting of K88ab, K88ac, K99 and 987P pili.

6. Combined vaccine according to claims 1-5, characterized in that the vaccine comprises immunogenic material of pseudorabies virus or bovine herpes virus.

7. Combined vaccine according to claims 1-6, characterized in that at least a portion of the viral material is pseudorabies virus or material with the immunogenic properties of pseudorabies virus.

8. Method for the preparation of a combined vaccine with immunizing action in animals against E. coli and herpes virus infection, characterized in that it is obtainable by combining immunogenic E. coli material which is rendered free of enzyme activity, and immunogenic herpes virus material.

9. Method according to claim 8, characterized in that E. coli material is rendered free of enzyme activity by at least one method selected from the group consisting of:
a. storing the E. coli material separately for a sufficiently long time for any proteolytic enzymes contained therein to become inactivated;
b. separating any proteolytic enzymes contained in the E. coli material from said material by differences in molecular size, electrical charge or bonding characteristics;
c. adding enzyme inhibitors;
d. denaturing the enzymes; and
e. adding protein material.

10. Method according to claim 9, characterized in that the vaccine is rendered free of enzyme activity by adding at least one enzyme inhibitor selected from the group consisting of N-ethyl maleimide, monoiodoacetate, monoiodoacetamide, Trasylol, ethylene diamine tetraacetic acid, phenylmethanesulphonyl fluoride and trypsin inhibitors.

11. Method according to claim 8, characterized in that at least one common component for the preparation of vaccines selected from the group consisting of:
a. stabilizers;
b. adjuvants;
c. buffering substances; and
d. preservatives, is added to the vaccine composition.

12. Method according to claim 8, characterized in that the E. coli material is rendered free of enzyme activity prior to being combined with the viral material.

13. Method according to claim 8, characterized in that the E. coli material is rendered free of enzyme activity during the combination with the viral material.

14. Method according to claim 8, characterized in that wherein the E. coli material is rendered free of enzyme activity after being combined with the viral material.

## Claims (Claims for the following Contracting State(s): ES)

1. Method for the preparation of a combined vaccine with immunizing action in animals against E. coli and herpes virus infection, characterized in that it is obtainable by combining immunogenic E. coli material which is rendered free of enzyme activity, and immunogenic herpes virus material.

2. Method according to claim 1, characterized in that E. coli material is rendered free of enzyme activity by at least one method selected from the group consisting of:
a. storing the E. coli material separately for a sufficiently long time for any proteolytic enzymes contained therein to become inactivated;
b. separating any proteolytic enzymes contained in the E. coli material from said material by differences in molecular size, electrical charge or bonding characteristics;
c. adding enzyme inhibitors;
d. denaturing the enzymes; and
e. adding protein material.

3. Method according to claim 2, characterized in that the vaccine is rendered free of enzyme activity by adding at least one enzyme inhibitor selected from the group consisting of N-ethyl maleimide, monoiodoacetate, monoiodoacetamide Trasylol, ethylene diamine tetraacetic acid, phenylmethanesulphonyl fluoride and trypsin inhibitors.

4. Method according to claim 1, characterized in that at least one common component for the preparation of vaccines selected from the group consisting of:
a. stabilizers;
b. adjuvants;
c. buffering substances; and
d. preservatives, is added to the vaccine composition.

5. Method according to claim 1, characterized in that the E. coli material is rendered free of enzyme activity prior to being combined with the viral material.

6. Method according to claim 1, characterized in that the E. coli material is rendered free of enzyme activity during the combination with the viral material.

7. Method according to claim 1, characterized in that the E. coli material is rendered free of enzyme activity after being combined with the viral material.

8. Method according to claim 1, characterized in that the immunogenic E. coli material is an isolated antigen fraction of E. coli.

9. Method according to claim 8, characterized in that the antigen fraction contains LT.

10. Method according to claim 8, characterized in that the antigen fraction contains pili.

11. Method according to claim 10, characterized in that one or more pili selected from the group consisting of K88ab, K88ac, K99 and 987P pili are used.

12. Method according to claims 1-11, characterized in that immunogenic material of pseudorabies virus or bovine herpes virus is used.

## Patentansprüche (Patentansprüche für folgende(n) Vertragsstaat(en): BE, CH, DE, FR, GB, IT, LI, NL, SE)

1. Kombiniertes Vakzin mit immunisierender Wirkung bei Tieren gegen E. coli- und Herpes-Infektion, erhältlich durch Kombination von E. coli-immunogenem Material, welches frei ist von Enzymaktivität, mit Herpesvirus immunogenem Material.

2. Kombiniertes Vakzin nach Anspruch 1, dadurch gekennzeichnet, dass das Vakzin eine isolierte Antigenfraktion von E. coli enthält.

3. Kombiniertes Vakzin nach Anspruch 2, dadurch gekennzeichnet, dass die Antigenfraktion LT enthält.

4. Kombiniertes Vakzin nach Anspruch 2, dadurch gekennzeichnet, dass die Antigenfraktion Pili enthält.

5. Kombiniertes Vakzin nach Anspruch 4, dadurch gekennzeichnet, dass das Vakzin ein oder mehrere Pili, ausgewählt aus der Gruppe bestehend aus K88ab-, K88ac-, K99- und 987P-Pili enthält.

6. Kombiniertes Vakzin nach den Ansprüchen 1 bis 5, dadurch gekennzeichnet, dass das Vakzin immunogenes Material des Pseudorabies-Virus oder des Rinderherpes-Virus enthält.

7. Kombiniertes Vakzin nach den Ansprüchen 1 bis 6, dadurch gekennzeichnet, dass mindestens ein Teil des viralen Materials Pseudorabies-Virus oder Material mit den immunogenen Eigenschaften des Pseudorabies-Virus ist.

8. Verfahren zur Herstellung eines kombinierten Vakzins mit immunisierender Wirkung bei Tieren gegen E. coli- und Herpesvirus-Infektion, dadurch gekennzeichnet, dass es erhältlich ist durch Kombination von immunogenem E. coli-Material, welches frei von Enzymaktivität gemacht ist, und immunogenem Herpes-Virus-Material.

9. Verfahren nach Anspruch 8, dadurch gekennzeichnet, dass das E. coli-Material frei von Enzymaktivität gemacht wird durch mindestens ein Verfahren, ausgewählt aus der Gruppe bestehend aus :
a. Lagerung des E. coli-Materials getrennt während genügend langer Zeit, dass alle darin enthaltenen proteolytischen Enzyme inaktiviert werden;
b. Abtrennung aller in dem E. coli-Material enthaltenen proteolytischem Enzyme von diesem Material auf Grund der Unterschiede in der Molekulargrösse, elektrischen Ladung oder Bindecharakteristiken;
c. Zusatz von Enzyminhibitoren;
d. Denaturierung der Enzyme und
e. Zusatz von Proteinmaterial.

10. Verfahren nach Anspruch 9, dadurch gekennzeichnet, dass das Vakzin frei von Enzymaktivität gemacht wird durch Zusatz von mindestens einem Enzmyinhibitor, ausgewählt aus der Gruppe bestehend aus N-Aethyl-maleinsäureimid-, Monojodacetat-, Monojodacetamid-, Trasylol-, Aethylendiamin-tetraessigsäure-, Phenylmethansulfonylfluorid- und Trypsin-Inhibitoren.

11. Verfahren nach Anspruch 8, dadurch gekennzeichnet, dass mindestens eine übliche Komponente für die Herstellung von Vakzinen, ausgewählt aus der Gruppe bestehend aus :
a. Stabilisatoren;
b. Adjuvantien;
c. Puffersubstanzen und
d. Konservierungsmitteln der Vakzinzusammensetzung zugesetzt wird.

12. Verfahren nach Anspruch 8, dadurch gekennzeichnet, dass das E. coli-Material frei von Enzymaktivität gemacht wird,bevor es mit dem viralen Material kombiniert wird.

13. Verfahren nach Anspruch 8, dadurch gekennzeichnet, dass das E. coli-Material frei von Enzymaktivität gemacht wird während der Kombination mit dem viralen Material.

14. Verfahren nach Anspruch 8, dadurch gekennzeichnet, dass das E. coli-Material frei von Enzymaktivität gemacht wird, nachdem es mit dem viralen Material kombiniert worden ist.

## Patentansprüche (Patentansprüche für folgende(n) Vertragsstaat(en): ES)

1. Verfahren für die Herstellung eines kombinierten Vakzins mit immunisierender Wirkung bei Tieren gegen E. coli- und Herpesvirus-Infektion, dadurch gekennzeichnet, dass es erhältlich ist durch Kombination von immunogenem E. coli-Material, welches frei von Enzymaktivität gemacht ist, mit immunogenem Herpesvirus-Material.

2. Verfahren nach Anspruch 1, dadurch gekennzeichnet, dass das E. coli-Material frei von Enzymaktivität gemacht wird durch mindestens ein Verfahren, ausgewählt aus der Gruppe bestehend aus :
a. Lagerung des E. coli-Materials getrennt während genügend langer Zeit, dass alle darin enthaltenen proteolytischen Enzyme inaktiviert werden;
b. Abtrennung aller in dem E. coli-Material auf Grund von Differenzen der Meolekülgrösse, elektrischen Ladung oder Bindecharakteristiken;
c. Zusatz von Enzyminhibitoren;
d. Denaturierung der Enzyme, und
e. Zusatz von Proteinmaterial.

3. Verfahren nach Anspruch 2, dadurch gekennzeichnet, dass das Vakzin frei von Enzymakivität gemacht wird durch Zusatz von mindestens einem Enzyminhibitor, ausgewählt aus der Gruppe bestehend aus N-Aethylmaleinsäureimid-, Monojodacetat-, Monojodacetamid-, Trasylol-, Aethylendiamin-tetraessigsäure-, Phenylmethansulfonylfluorid- und Trypsin-Inhibitoren.

4. Verfahren nach Anspruch 1, dadurch gekennzeichnet, dass mindestens eine übliche Komponente für die Herstellung von Vakzinen, ausgewählt aus der Gruppe bestehend aus :
a. Stabilisatoren;
b. Adjuvantien;
c. Puffersubstanzen und
d. Konservierungsmittel, zu der Vakzinzusammensetzung zugesetzt wird.

5. Verfahren nach Anspruch 1, dadurch gekennzeichnet, dass das E. coli-Material frei von Enzymaktivität gemacht wird bevor es mit dem viralen Material kombiniert wird.

6. Verfahren nach Anspruch 1, dadurch gekennzeichnet, dass das E. coli-Material frei von Enzymaktivität gemacht wird während der Kombination mit dem viralen Material.

7. Verfahren nach Anspruch 1, dadurch gekennzeichnet, dass das E. coli-Material frei von Enzymaktivität gemacht wird, nachdem es mit dem viralen Material kombiniert ist.

8. Verfahren nach Anspruch 1, dadurch gekennzeichnet, dass das immunogene E. coli-Material eine isolierte Antigenfraktion von E. coli ist.

9. Verfahren nach Anspruch 8, dadurch gekennzeichnet, dass die Antigenfraktion LT enthält.

10. Verfahren nach Anspruch 8, dadurch gekennzeichnet, dass die Antigenfraktion Pili enthält.

11. Verfahren nach Anspruch 10, dadurch gekennzeichnet, dass ein oder mehrere Pili, ausgewählt aus der Gruppe bestehend aus K88ab-, K88ac-, K99- und 987P-Pili verwendet werden.

12. Verfahren nach den Ansprüchen 1 bis 11, dadurch gekennzeichnet, dass immunogenes Material vom Pseudorabies-Virus oder Rinder-Herpes-Virus verwendet wird.

## Revendications (Revendications pour l'(les) Etat(s) contractant(s) suivant(s): BE, CH, DE, FR, GB, IT, LI, NL, SE)

1. Vaccin combiné présentant une activité immunisante chez l'animal, contre l'infection par E. Coli et par le virus de l'herpès, susceptible d'être obtenu en combinant du matériel immunogénique d'E. Coli, qui est exempt d'activité enzymatique, avec du matériel immunogénique du virus de l'herpès.

2. Vaccin combiné selon la revendication 1, caractérisé en ce que le vaccin contient une fraction antigénique d'E. Coli.

3. Vaccin combiné selon la revendication 2, caractérisé en ce que la fraction antigénique contient un antigène LT.

4. Vaccin combiné selon la revendication 2, caractérisé en ce que la fraction antigénique contient des pili.

5. Vaccin combiné selon la revendication 4, caractérisé en ce que le vaccin contient un ou plusieurs pili sélectionnés parmi le groupe de pili consistant en: K88ab, K88ac, K99 et 987P.

6. Vaccin combiné selon les revendications 1 à 5, caractérisé en ce que le vaccin comprend du matériel immunologique du virus de la pseudo-rage ou du virus de l'herpès bovin.

7. Vaccin combiné selon les revendications 1 à 6, caractérisé en ce qu'au moins une partie du matériel viral correspond soit au virus de la pseudo-rage soit à un matériel présentant les propriétés immunologiques du virus de la pseudorage.

8. Méthode de préparation d'un vaccin combiné, présentant une activité immunisante chez l'animal, contre une infection par E. Coli et par le virus de l'herpès, caractérisée en ce que son obtention se fait par combinaison de matériel immunogénique d'E. Coli, dont l'activité enzymatique a été éliminée (inactivée), et de matériel immunogénique du virus de l'herpès.

9. Méthode selon la revendication 8, caractérisée en ce que l'élimination de l'activité enzymatique du matériel d'E. Coli est réalisée par utilisation d'au moins un des procédés choisis parmi le groupe comprenant:
a) le stockage séparé du matériel d'E. Coli pendant un temps suffisamment long pour inactiver toutes les enzymes protéolytiques qu'il contient;
b) la séparation des enzymes protéolytiques contenues dans le matériel d'E. Coli, dudit matériel du fait de leur différence de taille, de charges électriques ou du fait de leurs caractéristiques de liaison;
c) l'addition d'inhibiteurs d'enzymes;
d) la dénaturation des enzymes; et
e) l'addition de matériel protéique.

10. Méthode selon la revendication 9, caractérisée en ce que l'activité enzymatique du vaccin est éliminée par addition d'au moins un des inhibiteurs d'enzymes, choisis parmi le groupe composé de: N-éthylmaléimide, monoiodoacétate, monoiodoacétamide, Trasylol, acide éthylènediamine tétraacétique, fluorure de phénylméthanesulphonyle et les inhibiteurs de trypsine.

11. Méthode selon la revendication 8, caractérisée en ce qu'au moins un constituant courant de la préparation de vaccin, choisi parmi le groupe composé de:
a) stabilisateurs;
b) adjuvants;
c) substances tampon; et
d) conservateurs (ou agents de protection), est ajouté à la composition du vaccin.

12. Méthode selon la revendication 8, caractérisée en ce que l'activité enzymatique du matériel d'E. Coli est éliminée avant l'étape de combinaison avec le matériel viral.

13. Méthode selon la revendication 8, caractérisée en ce que l'activité enzymatique du matériel d'E. Coli est éliminée pendant l'étape de combinaison avec le matériel viral.

14. Méthode selon la revendication 8, caractérisée en ce que l'activité enzymatique du matériel d'E. Coli est éliminée après l'étape de combinaison avec le matériel viral.

## Revendications (Revendications pour l'(les) Etat(s) contractant(s) suivant(s): ES)

1. Méthode de préparation d'un vaccin combiné, présentant une activité immunisante chez l'animal, contre l'infection par E. Coli et par le virus de l'herpès, caractérisée en ce qu'il est susceptible d'être obtenu par combinaison de matériel immunologique d'E. Coli, qui est exempt d'activité enzymatique, et de matériel immunologique du virus de l'herpès.

2. Méthode selon la revendication 1, caractérisée en ce que l'élimination de l'activité enzymatique du matériel d'E. Coli est réalisée par utilisation d'au moins un des procédés choisis parmi le groupe comprenant:
a) le stockage séparé du matériel d'E. Coli pendant un temps suffisamment long pour inactiver toutes les enzymes protéolytiques qu'il contient;
b) la séparation des enzymes protéolytiques contenues dans le matériel d'E. Coli, dudit matériel du fait de leur différence de taille, de charges électriques ou du fait de leurs caractéristiques de liaison;
c) l'addition d'inhibiteurs d'enzymes;
d) la dénaturation des enzymes; et
e) l'addition de matériel protéique.

3. Méthode selon la revendication 2, caractérisée en ce que l'activité enzymatique du vaccin est éliminée par addition d'au moins un des inhibiteurs d'enzymes, choisis parmi le groupe composé de: N-éthylmaléimide, monoiodoacétate, monoiodoacétamide, Trasylol, acide éthylènediamine tétraacétique, fluorure de phénylméthanesulphonyle et les inhibiteurs de trypsine.

4. Méthode selon la revendication 1, caractérisée en ce qu'au moins un constituant courant de la préparation de vaccin, choisi parmi le groupe composé de:
a) stabilisateurs;
b) adjuvants;
c) substances tampon; et
d) conservateurs (ou agents de protection), est ajouté à la composition du vaccin.

5. Méthode selon la revendication 1, caractérisée en ce que l'activité enzymatique du matériel d'E. Coli est éliminée avant l'étape de combinaison avec le matériel viral.

6. Méthode selon la revendication 1, caractérisée en ce que l'activité enzymatique du matériel d'E. Coli est éliminée pendant l'étape de combinaison avec le matériel viral.

7. Méthode selon la revendication 1, caractérisée en ce que l'activité enzymatique du matériel d'E. Coli est éliminée après l'étape de combinaison avec le matériel viral.

8. Méthode selon la revendication 1, caractérisée en ce que le vaccin contient une fraction antigénique d'E. Coli.

9. Méthode selon la revendication 8, caractérisée en ce que la fraction antigénique contient un antigène LT.

10. Méthode selon la revendication 8, caractérisée en ce que la fraction antigénique contient des pili.

11. Méthode selon la revendication 10, caractérisée en ce que le vaccin contient un ou plusieurs pili sélectionnés parmi le groupe de pili consistant en: K88ab, K88ac, K99 et 987P.

12. Méthode selon la revendication 1 à 11, caractérisée en ce que le vaccin comprend du matériel immunologique du virus de la pseudo-rage ou du virus de l'herpès bovin.
